# FASCICULE DE BREVET EUROPEEN

(11) **EP 2 938 188 B1**
(45) Date de publication et mention de la délivrance du brevet: **05.02.2020**
(21) Numéro de dépôt: 13830233.6
(22) Date de dépôt: 31.12.2013
(51) Int. Cl.: A01N 25/08, A01N 25/12, A01N 31/16, A01N 31/06, A01N 35/02, A01P 1/00, A61K 47/02, A61K 9/14

(54) **COMPLEXE MOLÉCULAIRE DÉTOXIFIANT ET ANTIMICROBIEN**
ANTIMIKROBIELLER MOLEKULARER ENTGIFTUNGSKOMPLEX
DETOXIFYING, ANTIMICROBIAL MOLECULAR COMPLEX

(30) Priorité: 31.12.2012 MA 35519
(43) Date de publication de la demande: 04.11.2015
(73) Titulaire: Remmal, Adnane, Fes 30 000 (MA)
(72) Inventeur: CHAMI, Ahmed Reda, 20200 Casablanca (MA)
(74) Mandataire: Cabinet Becker et Associés
(86) Numéro de dépôt international: PCT/MA2013/000054
(87) Numéro de publication internationale: WO 2014/104868

(56) Documents cités:
- EP-A1- 1 132 009
- WO-A2-2008/149232
- US-A1- 2013 230 431
- MARIE G.M. NGUEMTCHOUIN ET AL: "Ocimum gratissimum essential oil and modified montmorillonite clay, a means of controlling insect pests in stored products", JOURNAL OF STORED PRODUCTS RESEARCH, vol. 52, 1 janvier 2013 (2013-01-01), pages 57-62, XP55116327, ISSN: 0022-474X, DOI: 10.1016/j.jspr.2012.09.006
- SÉKOU MOUSSA KÉÏTA ET AL: "Insecticidal effects of Thuja occidentalis (Cupressaceae) essential oil on Callosobruchus maculatus [Coleoptera: Bruchidae]", CAN. J. PLANT SCI., 1 janvier 2000 (2000-01-01), XP55116352,
- Seâ Kou ET AL: "E?ect of various essential oils on Callosobruchus maculatus (F.) (Coleoptera: Bruchidae)", , 1 janvier 2000 (2000-01-01), XP55116354, Extrait de l'Internet: URL:http://ac.els-cdn.com/S0022474X9900055 7/1-s2.0-S0022474X99000557-main.pdf?_tid=f e8b5dec-d462-11e3-98eb-00000aab0f6c&acdnat =1399300919_e10c3be3b3513e86ba692857e8358a 23 [extrait le 2014-05-05]
- NGUEMTCHOUIN M M G ET AL: "Insecticidal formulation based on Xylopia aethiopica essential oil and kaolinite clay for maize protection", CROP PROTECTION, ELSEVIER SCIENCE, GB, vol. 29, no. 9, 1 septembre 2010 (2010-09-01), pages 985-991, XP027172020, ISSN: 0261-2194 [extrait le 2010-07-23]
- A. BRENES ET AL: "Essential oils in poultry nutrition: Main effects and modes of action", ANIMAL FEED SCIENCE AND TECHNOLOGY, vol. 158, no. 1-2, 1 juin 2010 (2010-06-01), pages 1-14, XP55116274, ISSN: 0377-8401, DOI: 10.1016/j.anifeedsci.2010.03.007
- Hojat Damiri ET AL: "Effect of Different Sodium Bentonite Levels on Performance, Carcass Traits and Passage Rate of Broilers", Pakistan Veterinary Journal, 1 mai 2012 (2012-05-01), pages 197-200, XP55116360, Extrait de l'Internet: URL:http://pvj.com.pk/pdf-files/32_2/197-2 00.pdf

## Description

La présente invention se rapporte à un additif pour aliment vétérinaire, une composition contenant cet additif ainsi qu'au procédé de fabrication de cette composition et à l'utilisation de cette composition en tant qu'additif à actions multiples: action de promoteur de croissance, plus l'action de protecteur contre les maladies parasitaires, plus l'action d'adsorbant de mycotoxines plus l'action immunostimulante et protecteur intestinal et hépatobiliaire.

Les pathologies qui touchent les animaux sont en général des phénomènes multifactoriels dans lesquels sont impliqués plusieurs éléments: bactéries, virus, champignons, parasites, immunodéficience, métabolisme, physiologie, environnement zootechnique, carences alimentaires, toxines et autre. L'éleveur dispose en général de produits variés qui traitent chacun un ou deux facteurs seulement. Il est donc obligé de composer des cocktails à partir de ces produits. Ceci à pour conséquence des risques d'inadéquation des traitements par excès ou par défaut générant des pertes diverses. La voie d'administration la plus utilisée pour ces traitements est la voie orale par addition dans l'aliment ou dans l'eau de boisson.

L'aliment de bétail contient entre autre des antibiotiques utilisés en tant que promoteurs de croissance et des agents antiparasitaires comme traitement préventif de la coccidiose ou de l'histomonose et d'autres maladies parasitaires. Ces agents chimio-thérapeutiques sont mis en cause dans plusieurs problèmes de santé animale et de santé publique pour leur toxicité, les résidus qu'ils peuvent générer dans les produits alimentaires d'origine animale et pour leur implication dans l'émergence de la résistance aux agents infectieux, Cháfer-Pericás et al, Food Control 22 (2011) 993-999.

C'est pour toutes ces raisons que ces agents ont été interdits dans plusieurs régions du monde. **IP/05/1687,** Bruxelles, 22 décembre 2005.

L'aliment de bétail contient aussi des concentrations non négligeables de mycotoxines provenant des moisissures qui contaminent les céréales contenus dans l'aliment de bétail. Ces mycotoxines ont des effets délétères pour la santé des animaux et leurs résidus dans les aliments d'origine animale sont délétères pour la santé du consommateur, Wayne and Bryden, Animal Feed Science and Technology 173 (2012) 134-158.

Dans ce contexte, les fabricants d'aliment de bétail et les éleveurs confrontés au problèmes de retard de croissance et des maladies parasitaires sont obligés de trouver des alternatives pour remplacer les antibiotiques utilisés comme promoteurs de croissance et les antiparasitaires qui ont été interdits comme additifs dans l'aliment. Ils doivent aussi trouver des additifs pour lutter contre les effets négatifs liés à la présence des moisissures qui produisent les mycotoxines et aux mycotoxines elles mêmes. (placinta et coll, A review of worldwide contamination of cereal grains and animal feed with Fusarium mycotoxins
Animal Feed Science and Technology Volume 78, Issue 1 , Pages 21-37, 31 March 1999)

Pour empêcher ces mycotoxines de passer dans la circulation, plusieurs moyens sont utilisés. Le moyen le plus utilisé est l'usage d'adsorbants tels que : Hydrated Sodium Calcium Alimunosilicate (HSCAS), charbon activé, parois de levures, Phyllosilicates, Cholestyramine, bactéries lactiques, Phillips, Clement & Park, 1994. Approaches to reduction of aflatoxins in foods and feeds. *In* D. L. Eaton & J.D. Groopman, eds. The toxicology ofaflatoxins - human health, veterinary and agricultural significance, p. 383. San Diego, California, United States, Academic Press.

Parmi les adsorbants on trouve les argiles activés par addition d'acides et chauffage, EP 1 333 919 B1, UTILISATION DE SILICATES EN COUCHES ACTIVES POUR L'ADSORPTION DE MYCOTOXINES. Cependant, les procédés d'activation sont très compliqués et très coûteux, ce qui se répercute négativement sur la qualité et la reproductibilité de la capacité d'adsorption des mycotoxines et rend le prix des argiles trop cher surtout quand il s'agit de l'aliment de bétail.

Un autre problème lié aux argiles activées consiste dans le fait que ces argiles peuvent agir en chélateurs de vitamines et diminuer la quantité de vitamines disponibles pour l'animal. Ceci peut engendrer des problèmes de carences vitaminiques.

Parmi les alternatives utilisées pour remplacer les antibiotiques et les antiparasitaires on trouve les huiles essentielles et leurs composés majoritaires tels que thymol, carvacrol, cynnamaldehyde, eugénol et autres (Alleman et coll, Utilisation des huiles essentielles en alimentation des volailles. INRA Productions Animales, 2013, numéro 1 ; Bento et coll, Essential oils and their use in animal feeds for monogastric animais, Veterinarni Medicina, 58, 2013 (9): 449-458; Brenes and Roura Animal Feed Science and Technology 158 (2010) 1-14).

Cependant, ces substances sont volatiles et donc instables dans l'aliment. Cette instabilité est à l'origine d'une perte importante des huiles essentielles au cours de la fabrication de l'aliment. Ceci oblige les fabricants d'aliment de bétail à ajouter de grandes quantités d'huiles essentielles en excès pour que l'aliment une fois fabriqué contienne encore la quantité nécessaire d'huiles essentielles ou à utiliser des méthodes de stabilisation compliquées et onéreuses telles que la micro-encapsulation (de Barros Fernandes et coll, Gum arabic/starch/maltodextrin/inulin as wall materials on the microencapsulation of rosemary essential oil, Carbohydrate Polymers 101 (2014) 524-532).

Cette instabilité est aussi à l'origine de la faible durée de validité des actions recherchés dans l'aliment de bétail après fabrication. Ceci se répercute négativement sur la qualité de l'aliment contenant les huiles essentielles comme additif et augmente le prix de l'additif, ce qui diminue sa compétitivité face aux antibiotiques et aux antiparasitaires et aux autres alternatives.

Dans l'art antérieur, il a été décrit des compositions comprenant de l'argile et des composés volatiles pour lutter contre l'humidité et les odeurs nauséabondes (cf. la demande de brevet US 2013-230431, document appartenant à l'état de l'art selon l'article 54(3) CBE), des additifs alimentaires comprenant un composé actif obtenu à partir de plantes du genre *Citrus* et un support minéral poreux (cf. la demande de brevet EP 1 132 009), et des formulations insecticides comprenant une huile essentielle d*'Ocimum gratissimum* et de la montmorillonite (Nguemtchouin et al., Journal of Stored Products Research, vol. 52, 2013, 57-62) ou comprenant de l'huile essentielle de *Thuja occidentalis, Tagetes minuta, Hyptis suaveolens, Ocimum canum, Ocimum basilicum, Piper guineense* ou *Xylopia aethiopica* et du kaolin (Sékou Moussa Kéïta et al., Revue canadienne de phytotechnie, 2001, 81(1): 173-177 ; Sékou Moussa Kéïta et al., Journal of Stored Products Research, vol. 36, 2000, 355-364 ; Nguemtchouin et al., Crop Protection, vol. 29, 2010, 985-991). Les effets des huiles essentielles ou du bentonite de sodium sur les poulets d'élevage ont également été décrits (Brenes and Roura, Animal Feed Science and Technology, 158, 2010, 1-14 ; Damiri et al., Pak. Vet. J., 2012, 32(2) : 197-200).

L'objet de la présente invention est défini par les revendications.

Le but de la présente invention consiste à inventer une composition stable contenant des composés ayant une activité antibiotique (antibactérienne) et antiparasitaire, une activité anti-moisissure capable d'inhiber la croissance des moisissures secrétant les mycotoxines et en même temps la capacité d'adsorber les mycotoxines. Un autre but de l'invention consiste à sélectionner parmi les substances ayant les activités citées ci-dessus, celles qui ont aussi une activité de protecteur intestinal et hépato-biliaire et d'immunostimulant.

Ainsi, contre les pathologies qui sont des phénomènes multifactoriels affectant la santé animale l'invention propose une solution à actions multiples.

La composition objet de la présente invention et visant à atteindre le but précité, résulte de la complexassion par intercalation de molécules volatiles telles que: thymol, crésol, carvacrol, menthol, eugenol, cynnamaldehyde, ayant une activité antibactérienne, antiparasitaire, antifongique et protecteur intestinal, hépatobiliaire et immunostimulant avec des argiles. L'argile ayant en même temps l'action d'excipient stabilisateur de la composition, l'action d'adsorbant de mycotoxines et l'action de protecteurs intestinal.

L'expression complexe moléculaire désigne toute composition obtenue par mélange des huiles essentielles ou l'un de leurs composés volatils avec toute argile comestible dont les molécules sont capables d'interagir avec ces huiles essentielles et leurs composés volatils.

L'expression huiles essentielles désigne tout extrait obtenu à partir de plantes aromatiques par l'une des méthodes d'extraction connues permettant de les extraire.

L'expression composés volatils des huiles essentielles désigne toute molécule volatile obtenue par purification à partir des huiles essentielles ou d'une autre source naturelle ou alors par synthèse chimique à l'identique ainsi que leurs dérivés et isomères.

L'expression argile comestible désigne toute substance minérale naturelle ou synthétique ayant une structure moléculaire identique ou similaire à celle des argiles comestibles communément utilisées dans le domaine agro-alimentaire et pharmaceutique.

L'expression argile intercalée désigne toute situation ou des molécules intercalantes entrent en interaction avec les molécules d'argile en s'insérant dans les espaces interfoliaires de l'argile. Cette intercalation se traduisant par une augmentation de la distance interfoliaire.

L'expression activité antimicrobienne désigne toute action inhibitrice ou destructrice des germes microbiens de nature bactérienne, fongique, parasitaire ou virale.

L'expression protecteur intestinal et hépatobiliaire désigne toute action permettant de préserver l'intégrité physique et physiologique de l'intestin et de stimuler la fonction hépatique pour augmenter son activité de détoxification.

L'expression immunostimulant désigne toute activité permettant de renforcer les défenses naturelles ou acquises de l'organisme.

L'expression adsorbant de toxines désigne toute activité permettant de fixer les toxines au niveau intestinal pour empêcher leur absorption vers la circulation sanguine.

L'élevage animal est confronté à une multitude de facteurs de risque d'ordre zootechnique, infectieux, sanitaire, hygiénique, immunitaire, physiologique et alimentaire qui favorisent l'apparition de pathologies qu'un seul de ces facteurs ne pourrait pas provoquer. C'est pour cette raison que les scientifiques s'accordent sur l'idée que l'apparition de toute maladie est un phénomène multifactoriel (Sjaak de Wit, Practical epidemiology of poultry disease and multifactorial conditions, Poultry Diseases (Sixth Edition), 2008, Pages 492-509).

D'où l'intérêt d'étudier l'impact de chaque facteur à part et d'apporter un remède curatif ou préventif à chacun pour empêcher l'apparition de la maladie ou atténuer les dégâts qu'elle provoque. La multitude de remèdes pose un problème de coût, de dosage de chaque remède et bien d'autres problèmes qui compliquent le travail de l'éleveur.

Pour palier au problème de l'usage d'antibiotiques et d'antiparasitaires de chimiothérapie interdits par la réglementation et au problème de remèdes multiples, la présente invention a par conséquent pour objet une composition stable constituée d'un complexe moléculaire contenant des molécules naturelles possédant des activités pharmacologiques multiples; antimicrobienne, immunostimulantes, cholagogue, carminative telles que le thymol, le crésol, le carvacrol, le menthol, l'eugénol, le cinnamaldéhyde, ou tout composant les contenant séparément ou mélangés ainsi que les isomères, dérivés et mélanges de ceux-ci. Ces molécules volatils sont fixées et stabilisées par intercalation dans l'espace interfoliaire d'argiles possédant des propriétés adsorbants de toxines, protecteurs intestinaux, et hydratantes composées de stevensite et de bentonite, à savoir des mélanges provoqués ou naturellement interstratifiés.

Dans un mode de réalisation particulier, la composition peut comprendre les dits agents antimicrobiens volatils qui sont des alcools aromatiques antiseptiques choisis parmi le thymol, le crésol, le carvacrol, le menthol, l'eugénol, le cinnamaldehyde, ou tout composant les contenant séparément ou mélangés ainsi que les isomères, dérivés et mélanges de ceux-ci. Ces agents sont intercalés dans une argile comestible qui est un mélange de stevensite et de bentonite provoqué ou naturellement interstratifié.

Le dit agent antimicrobien volatil est présent dans la dite composition dans un ratio en poids allant de 0,005 à 0,33 par rapport à la dite argile comestible.

Le procédé de fabrication de la dite composition comprend les étapes suivantes:
i/ Le dit agent antimicrobien volatil est mis en solution dans un solvant organique ou minéral pour obtenir la solution antimicrobienne.
ii/ La dite solution antimicrobienne ainsi obtenue est mélangée avec la dite argile comestible sous forme de poudre ou de pellets dans des conditions d'agitation et de température permettant d'obtenir une composition homogène et stable constituée par un complexe moléculaire contenant la dite argile comestible intercalée par le dit agent antimicrobien volatil.

Dans un mode de réalisation particulier, ce procédé de fabrication peut être détaillé comme suit:
- Une quantité d'agent antimicrobien volatil est mise progressivement en solution dans une quantité d'huile végétale ou minérale, représentant 1 à 20% de la quantité d'agent antimicrobien volatil, agitée et chauffée à une température allant de 30 à 80°C pour obtenir une solution liquide homogène et limpide dite solution antimicrobienne.
- Une quantité de la solution antimicrobienne est inclue progressivement par mélange dans une quantité de la dite argile comestible en poudre, représentant au moins 3 fois la quantité de solution antimicrobienne, pour obtenir une poudre tamisable.
- La poudre tamisable obtenue est calibrée par tamisage pour obtenir la granulométrie désirée.

Dans un mode de réalisation particulier, l'agent antimicrobien volatil peut être le thymol seul ou mélangé avec un ou plusieurs autres agents antimicrobiens volatils comme le crésol, l'eugénol, le carvacrol, le cinéol, le menthol ou le cynnamaldéhyde ou toute huile essentielle les contenant séparément ou mélangés et l'argile comestible est un mélange de stevensite et de bentonite ou le mélange de substances les contenants.

Dans un mode de réalisation préféré, les préparations telles que détaillées ci-avant seront destinées à être dilués dans l'aliment de bétail pour administration à un animal. Cette dilution peut être effectuée dans un rapport allant de 50 grammes de préparation par tonne d'aliment à 10 kilogrammes de préparation par tonne d'aliment.

Dans encore un mode de réalisation préféré la dite préparation, avant dilution dans l'aliment, peut contenir l'agent antimicrobien volatil à un ratio en poids allant de 0,1 à 0,33 par rapport à l'argile comestible. Préférentiellement dans un ratio en poids de 0,15 à 0,2 par rapport à l'argile.

La présente description décrit l'utilisation des dites compositions comme additif alimentaire chez l'animal ajoutées à l'aliment et prises par voie orale.

Dans tous les cas l'utilisation des dites compositions en tant qu'additifs alimentaires destinés à exercer une multitude d'actions émanant des propriétés des substances qu'elles contiennent à savoir :
- l'activité promoteur de croissance due aux propriétés antiseptiques des agents antiseptiques volatils capables de diminuer la flore intestinale pathogène ou commensale.
- l'activité antiparasitaire, anticoccidienne et antihistomonose, en particulier due à l'activité antiparasitaire des agents antimicrobiens volatils.
- l'activité antifongique due à l'activité antifongique des agents antimicrobiens volatils permettant d'inhiber les moisissures responsables de la production de mycotoxines.
- l'activité adsorbant de mycotoxines due aux propriétés de chélateurs des argiles.
- l'activité cholagogue due aux propriétés pharmacologiques des agents antimicrobiens volatils.
- l'activité immunostimulante due aux propriétés pharmacologiques des agents antimicrobiens volatils.
- L'activité de protecteur intestinal due à la fois aux propriétés de pansement intestinal de l'argile et aux propriétés antiseptiques intestinal des agents antimicrobiens volatils.

Dans tous les cas l'utilisation des dites compositions en tant qu'additifs alimentaires destinés à exercer une multitude d'actions favorables à l'organisme animal ayant pour conséquence la préservation de l'intégrité intestinale, hépatobiliaire et immunitaire avec des répercutions positives sur la préservation de la santé générale des animaux recevant les dites composition comme additifs alimentaires.

Les exemples qui suivent sont destinés à illustrer certains aspects de l'invention sans toutefois être limitatifs.

D'une part, l'inventeur a procédé à des expériences avec différents types d'agents antimicrobiens volatils et différents types d'argiles. Les résultats ont permis de mettre en évidence un déplacement des pics des diffraction aux rayons X des molécules caractéristiques des argiles testées à savoir la stevensite et la bentonite dans un sens qui montre une augmentation de la distance interfoliaire des argiles intercalées par les agents antimicrobiens volatils comparées aux argiles avant intercalation. Ceci justifie et explique la fixation parfaitement stable des agents antimicrobiens volatils sur les argiles quand ils sont mélangés selon le procédé sujet de l'invention. D'autre part, l'inventeur a testé l'efficacité de la composition avec les doses et les ratios particulièrement efficaces de la composition dans des conditions *in vitro* et *in vivo :*

### Exemple 1 : Essai de diffraction aux rayons X

Des préparations d'un poids final de 1000 grammes ont été réalisées avec 150 grammes de thymol mis en solution selon le procédé de l'invention avec 20 millilitres d'huile de table à une température de 60°C. La solution antiseptique ainsi obtenus a été mélangée avec 830 grammes de ghassoule du Maroc ou avec 830 grammes de bentonite du Maroc ou avec830 grammes d'un mélange 50%/50% ghassoul/bentonite. (préparation a)

Des compositions témoins ont été préparées :
Préparation b/ Les mêmes argiles pures sans aucun additif
Préparation c/ Les mêmes argiles avec 20ml d'huile de table seule
Préparation d/ les mêmes argiles avec le thymol seul en poudre cristallisée
Préparation e/ Les mêmes argiles avec du thymol liquéfié par chauffage à 60°C sans huile végétale.

Les préparations a, b et c ont été soumises à un test de diffraction aux rayons X selon les normes habituels de traitement des argiles dans cette méthode analytique.

Les résultats obtenus montrent un déplacement du pic spécifique de la bentonite et de la steveniste dans le spectre de façon étonnante dans le cas des préparations selon le procédé de l'invention (préparation a) comparées au témoin des argiles pures (préparation b) et des argiles ayant été mélangés à l'huile de table seule (préparation c) (résultats tableau 1). En même temps les résultats montrent que pour la préparation (d) contenant du thymol en poudre cristallisée mélangé à l'argile, la poudre obtenue est hétérogène et le spectre de diffraction aux rayons X obtenu était inexploitable. Pour la préparation (e) dans laquelle le thymol a été liquéfié par chauffage et mélangé à l'argile sans huile végétale, on voit que le thymol se re-cristallise et forme des grumeaux dure. Le mélange ainsi obtenu est hétérogène et ne peux répondre aux critères pour lesquels l'invention a été faite.

Ceci montre d'une part que le procédé de fabrication utilisé dans l'invention permet effectivement d'aboutir à l'intercalation du thymol dans l'espace interfoliaire des argiles, ce qui se traduit par une dispersion stable et homogène du thymol dans l'argile et à une augmentation de la distance de l'espace interfoliaire des argile améliorant par conséquent l'hydrophobicité et la capacité des argiles à adsorber les toxines.

Ces résultats montrent d'autre part, que les mélanges entre thymol et argile par les méthodes évidentes utilisant le thymol cristallisé ou liquéfié par chauffage n'aboutissent pas au résultat attendu par l'invention.

**Tableau 1 : Distance interfoliaire des argiles selon les modes de préparation a, b et c.**

| Les argiles | Bentonite | | | Steveniste | | |
|---|---|---|---|---|---|---|
| | Brute (prep b) | Avec huile de table (prep c) | Avec huile de table et huile essentielle (prep a) | Brute (prep b) | Avec huiles de table (prep c) | Avec huile de table et huile essentielle (prep a) |
| La distance entre les feuillets en (Angstrom) | 15.23 | 15.23 | 15.69 | 15.75 | 16.15 | 16.51 |

### Exemple 2: Essai in vivo

Des lots de 25 poussins de poulet de chair ainsi que douze dindonneaux ont été nourrit par un aliment contenant la préparation de l'invention à raison de 100 grammes, 500 grammes, 1kilogramme ou 2kilogrammes part tonne d'aliment. Des lots témoins de la même taille ont été nourrit par un aliment ne contenant aucun antibiotique ni antiparasitaire. D'autres lots témoins de la même taille ont été nourrit avec un aliment contenant la flavomycine à raison de 200 grammes par tonne d'aliment et la Salynomicine à raison de 70 grammes par tonne d'aliment pour le poulet de chair et 100 grammes par tonne de monensin pour les dindonnaux. Durant cinq semaines de traitement, des prélèvements de fèces de chaque sujet dans chaque lot ont été analysés par microscopie et par des analyses microbiologiques appropriés pour évaluer le nombre de bactéries, de levure, de moisissures et d'ookystes de protozoaires en particulier de l'espèce *eimeria. sp* impliquée dans la coccidiose chez le poulet et la dinde. A la fin des cinq semaines de l'expérience les sujets ont été sacrifiés et une autopsie a été effectuée par des vétérinaires professionnels spécialistes en pathologie aviaires. Le but de l'autopsie était d'évaluer les scores lésionnels au niveau de l'intestin et l'état d'intégrité du foie, des reins, des poumons et de la rate et de l'intestin.

Les résultats obtenus montrent des diminutions très significatives du nombre de bactéries, de levures, des moisissures et des ookystes chez les lots ayant reçu la préparation de l'invention dans leur aliment aux doses de 1 kilogramme par tonne et deux kilogrammes par tonne par rapport aux lots témoins ayant reçu un aliment neutre. Les lots ayant reçu la préparation de l'invention dans l'aliment aux doses de 100 grammes et 500 grammes par tonne d'aliment ne présentent pas de différence significative par rapport au témoin. Les lots ayant reçu l'antibiotique et l' antiparasitaire dans l'aliment présente une diminution visible mais non significative par rapport au témoin. Cependant, le nombre de bactéries, de levure, de moisissures et d'ookystes chez les lots ayant reçu la préparation de l'invention aux doses de 1 kilogramme et deux kilogrammes par tonne d'aliment est significativement inférieur au nombre de bactéries, de levures, de moisissure et d'ookystes enregistré dans les lots ayant reçu l'antibiotique et l'antiparasitaire dans l'aliment. Ces résultats montrent que la préparation selon l'invention utilisée comme additif dans l'aliment de poulet et de dinde possède des activités multiples comme promoteur de croissance (réduction de la flore intestinale), comme agent antiparasitaire (anticoccidien) et agent antifongique de façon significativement supérieur aux antibiotiques et antiparasitaires utilisés dans cet essai. Les résultats de l'autopsie aussi montrent que les scores de lésions intestinales, de congestion des reins, des poumons, de la rate et du foie sont significativement plus bas chez les lots ayant reçu l'aliment contenant la préparation de l'invention par rapport aux lots ayant reçu l'aliment neutre ou l'aliment contenant l'antibiotique et l'antiparasitaire. Ces résultats montrent encore que la préparation de l'invention utilisée comme additif dans l'aliment de poulet et de dinde possède des activités de protecteur de l'intégrité intestinale, de protecteur hépatobiliaire, de purificateur des poumons et des reins et de protecteur de la rate. Ces résultats confirment que la préparation de l'invention représente une solution à activités multiples pour lutter contre les pathologies du poulet et de la dinde qui sont des maladies multifactorielles.

Des lots de 25 poussins d'un jour ont été nourris pendant 5 semaines en moyenne avec respectivement :
- lot 1 : un aliment « poulets de chair » blanc qui ne contient ni des antibiotiques ni des antiparasitaire ;
- lot 2 : un aliment contenant un antibiotique facteur de croissance « Flavomicine » à raison de 200 milligramme par Kilogramme d'aliment et un antiparasitaire « Salynomicine » à raison de 70 milligramme par Kilogramme;
- lot 3 : un aliment contenant la préparation de l'invention dans l'aliment à une proportion de 100 grammes par tonne d'aliment;
- lot 4 : un aliment contenant la préparation de l'invention dans l'aliment à une proportion de 500 grammes par tonne d'aliment;
- lot 5 : un aliment contenant la préparation de l'invention dans l'aliment à une proportion de 1 Kg par tonne d'aliment;
- lot 6 : un aliment contenant la préparation de l'invention dans l'aliment à une proportion de 2 Kg par tonne d'aliment;

**Tableau 2: Résultats zootechniques et microbiologiques de l'essai in vivo poulet de chair**

| | ***Taux de mortalité*** | ***Indice de consommation*** | ***Le poids corporel (g)*** | ***La charge bactérienne* (UFC/g de fèces)** | ***La charge fongique* (UFC/g de fèces)** |
|---|---|---|---|---|---|
| **Lot 1** | 0% | 2,3 | 105 *±* 8,6 | 1.10¹⁰ *±* 0,3 | 8,6.10⁹ *±* 3,7 |
| **Lot 2** | 0% | 2,2 | 110 *±* 6,4 | 4,9.10⁹ *±* 2,1 | 1,6.10⁹ *±* 0,3 |
| **Lot 3** | 0% | 2,2 | 113 *±* 3,4 | 4,510⁹ *±* 3,2 | 5,7.10⁸ *±* 1,2 |
| **Lot 4** | 0% | 2,1 | 121 *±* 4,5 | 3,2.10⁸ *±* 4,1 | 1,3.10⁸ *±* 0,3 |
| **Lot 5** | 0% | 1,9 | 138 *±* 6,6 | 7,3.10⁷ *±* 5,2 | 8,6.10⁷ *±* 1,5 |
| **Lot 6** | 0% | 1,8 | 157 *±* 2,2 | 2.10⁷ *±* 1,70 | 5.10⁷ *±* 1,1 |

L'essai in vivo pour la dinde a donné des résultats similaires à ceux obtenus dans l'essai *in vivo* poulet de chair.

### Exemple 3 : Essais terrain

Des essais térrain ont été effectués dans une ferme d'élevage de poulet de chair et une ferme d'élevage de dinde de chair. Dans ces essais, deux lots de 13500 poussins chacun ont reçu la préparation de l'invention à la dose de deux kilogrammes par tonne d'aliment durant toute la durée de la bande (40 jours pour le poulet et 13 semaines pour la dinde). Deux autres lots de 13500 poussins chacun ont reçu la préparation de l'invention à la dose de 1 kilogramme par tonne d'aliment et deux autres lots de 13500 poussins chacun ont reçu un aliment contenant l'antibiotique flavomycine à 200 grammes par tonne d'aliment et l' antiparasitaire salynomicine à 70 grammes par tonne d'aliment pour le poulet et du monensin pour la dinde. A la fin de la bande, les performances zootechniques et les scores lésionnels ont été mesurés pour chaque lot.

Les résultats montrent :
La mortalité : le pourcentage de poulets et de dindes morts durant le cycle d'élevage était de 2,9% pour le poulet et 2,5 % pour la dinde au niveau des lots ayant reçu 1kilogramme ou 2 kilogramme de préparation de l'invention par tonne d'aliment. Au niveau des lots ayant reçu l'antibiotique et l'antiparasitaire dans l'aliment, la mortalité était de 5,2% pour la dinde et 5,9% pour le poulet.
Le poids moyen : Le poids moyen des deux lots ayan reçu 1 kilogramme ou deux kilogrammes par tonne d'aliment de la préparation de l'invention est de 2,3 kg par sujet vendu chez le poulet et 12,8 Kg chez la dinde, celui des deux lots ayant reçu l'antibiotique et l'antiparasitaire était de 2,135 kg chez le poulet et 11,9 Kg chez la dinde.
L'indice de consommation : pour les lots ayant reçu deux kilogramme de la préparation de l'invention par tonne d'aliment était de 1,68, celui des lots ayant reçu un kilogramme de la préparation par tonne d'aliment était de 1,82, celui des deux lots ayant reçu l'antibiotique et l'antiparasitaire était de l'ordre de 1,98.
Les titres des anticorps relatifs aux vaccins utilisés : titre inhibition de l'hémagglutination par rapport aux anticorps du virus de la maladie de Newcastle : les lots ayant reçu la préparation à raison de deux kilogrammes et un kilogramme par tonne d'aliment étaient respectivement 64, 64, 64, 32 et pour les deux lots ayant reçu l'antibiotique et l'anticoccidien ils étaient de 16 et 16.
Le score lésionnel de l'intestin : Les scores lésionnels de l'intestin était au plus bas (1,22) pour les lots ayant reçu la préparation de l'invention à raison de deux kilogramme et un kilogramme alors que les score pour les lots ayant reçu l'antibiotique et l'anticoccidien était de l'ordre de 2,74 et 3,13.
Le score de l'intégrité des organes : l'aspect des organes (foie, rate, poumons, muscles et os) était nettement plus sains (ne présentant aucun signe de congestion) pour les lots ayant reçu la préparation de l'invention à raison de deux kilogrammes et un kilogramme par tonne d'aliment alors que des signe de congestion visibles à l'œil nu était constatés chez la majorité des sujets autopsiés dans les deux lots ayant reçu l'antibiotique et l'antiparasitaire.

Ces résultats montrent d'une façon étonnante que la préparation de l'invention exerce les activités multiples pour lesquelles elle a été conçu et ce même dans les conditions d'élevage intensif sur le terrain.

### Exemple 4 : Inhibition et adsorption des mycotoxines dans l'aliment et le maïs :

Des échantillons de maïs et d'aliment composé ont été traités avec des quantités variables de la préparation de l'invention dans les proportions de 2 kilogramme, 4 kilogrammes et 6 kilogrammes par tonne d'aliment. Ces échantillons ont été emballés hermétiquement et incubées à une température de 27,5 °C. Des aliquotes de 50 grammes ont été prélevés après quatre semaines pour un dosage des mycotoxines : Les résultats montrent que les échantillons de Maïs ayant été traités par la préparation de l'invention présente un taux de DON (déoxynivalenol) de ZON (Zearalenone) et d'ochratoxine significativement plus bas que l'échantillon témoin. Cet effet d'inhibition et d'adsorption des mycotoxine et croissant en fonction de la dose de préparation de l'invention utilisée. Un résultat similaire a été obtenu pour l'échantillon d'aliment composé traité par la préparation de l'invention par rapport à l'échantillon non traité et ce, pour les mycotoxines Ochratoxine (ZON) et Aflatoxine.

Ces résultats montrent que la préparation de l'invention possède aussi la capacité de protéger l'aliment et le maïs des mycotoxines qui représentent un facteur de risque important favorisant l'apparition des maladies et provoquant dans les cas aigue des intoxications mortelles pour les animaux.

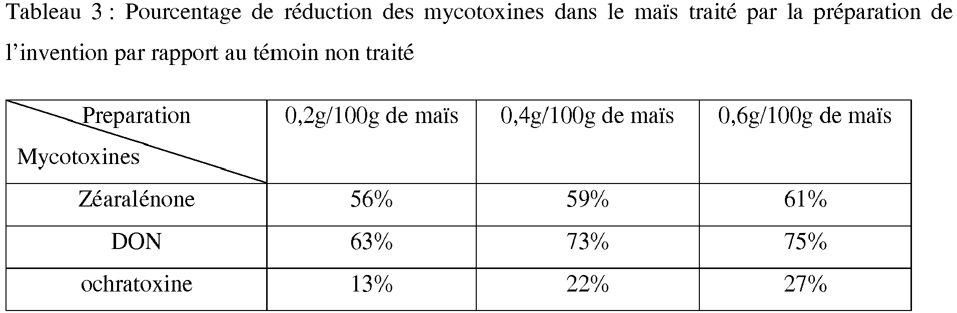

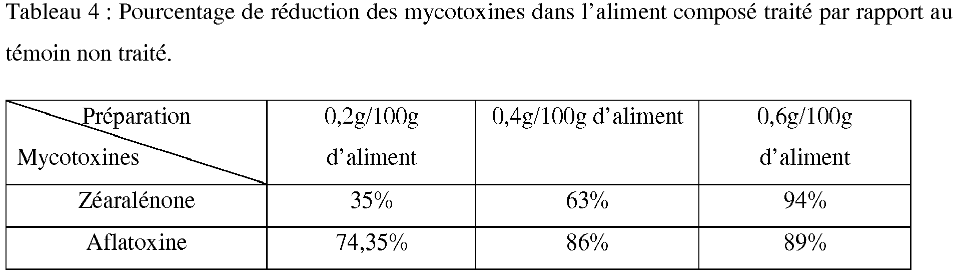

## Revendications

1. Composition combinant à la fois les activités antimicrobienne et d'adsorbant de toxines **caractérisée en ce qu'**elle est constituée par un complexe moléculaire obtenu par mélange d'un agent antimicrobien volatil avec une argile comestible, ledit agent antimicrobien volatil étant intercalé dans l'espace interfoliaire de l'argile et ladite argile étant un mélange de stevensite et de bentonite provoqué ou naturellement interstratifié.

2. Composition selon la revendication 1 **caractérisée en ce que** ledit agent antimicrobien est une huile essentielle ou l'un de ses composés volatils, de préférence un alcool aromatique antiseptique choisi parmi le thymol, le crésol, le carvacrol, l'eugénol, le menthol et le cinnamaldéhyde, naturels ou synthétiques, et les isomères, dérivés et mélanges de ceux-ci.

3. Composition selon l'une quelconque des revendications précédentes **caractérisée en ce que** ledit agent antimicrobien volatil est constitué par un mélange de deux ou plusieurs des agents antimicrobiens naturels ou synthétiques.

4. Composition selon l'une quelconque des revendications précédentes **caractérisée en ce que** ladite argile comestible est un mélange de Ghassoul et de bentonite, de préférence un mélange de Ghassoul du Maroc et de bentonite du Maroc, consistant de préférence pour au moins 5 % en poids, et de manière plus particulièrement préférée pour au moins 30 % en poids, de Ghassoul du Maroc.

5. Composition selon l'une quelconque des revendications précédentes **caractérisée en ce que** le dit agent antimicrobien volatil est présent dans un ratio massique de 0,005 à 0,33 par rapport à la dite argile comestible.

6. Procédé de fabrication de ladite composition selon les revendications 1 à 5 **caractérisé en ce qu'**il comprend les étapes suivantes:
i. Ledit agent antimicrobien volatil est mis en solution dans un solvant organique ou minéral pour obtenir la solution antimicrobienne,
ii. Ladite solution antimicrobienne ainsi obtenue est mélangée avec ladite argile comestible sous forme de poudre ou de pellets dans des conditions d'agitation et de température permettant d'obtenir une composition homogène et stable constituée par un complexe moléculaire contenant ladite argile comestible intercalée par ledit agent antimicrobien volatil,
de préférence, ledit agent antimicrobien volatil est choisi parmi le thymol, le crésol, le carvacrol, le menthol, l'eugénol et le cinnamaldéhyde, et les isomères, dérivés et mélanges de ceux-ci, ledit solvant organique ou minéral est une huile végétale et ladite argile comestible est un mélange de steveniste et de bentonite provoqué ou naturellement interstratifié.

7. Procédé de fabrication selon la revendication 6 **caractérisé en ce que** :
i. Une quantité d'agent antimicrobien volatil est mise progressivement en solution dans une quantité d'huile végétale, représentant 1 à 20% de la quantité d'agent antimicrobien volatil , agitée et chauffée à une température allant de 30 à 80°C pour obtenir une solution liquide homogène et limpide dite solution antimicrobienne ;
ii. Une quantité de la solution antimicrobienne est inclue progressivement par mélange dans une quantité de ladite argile comestible en poudre, représentant au moins 3 fois la quantité de solution antimicrobienne, pour obtenir une poudre tamisable ;
iii. La poudre tamisable obtenue est calibrée par tamisage pour obtenir la granulométrie désirée.

8. Procédé de fabrication selon la revendication 6 ou 7 **caractérisé en ce que** ledit agent antimicrobien est le thymol et ledit solvant organique est une huile végétale, et ladite argile comestible est le Ghassoul du Maroc seul ou mélangé avec la Bentonite du Maroc.

9. Procédé de fabrication selon la revendication 8 **caractérisé en ce que** l'étape de mise en solution progressive d'une quantité de thymol dans une quantité d'huile végétale représentant 1 à 20% de la quantité de thymol se fait à une température allant de 40 à 60°C.

10. Composition selon l'une quelconque des revendications 1 à 5, ladite composition étant un additif alimentaire pour un animal.

11. Composition selon la revendication 10, dans laquelle ledit animal est choisi parmi les mammifères, de préférence choisis parmi les bovins, les ovins, les lapins, les porcs, les caprins et les équidés, les poissons, les abeilles et les volailles, de préférence choisies parmi le poulet de chair, la poule pondeuse, les coqs et poules reproducteurs, la pintade, le dindon, la caille, le canard, l'oie et le pigeon.

## Patentansprüche

1. Zusammensetzung, die gleichzeitig antimikrobielle und Toxin-bindende Aktivitäten vereint, **dadurch gekennzeichnet, dass** sie aus einem Molekülkomplex besteht, der durch Mischen eines flüchtigen antimikrobiellen Mittels mit einer essbaren Tonerde erhalten wird, wobei besagtes flüchtiges antimikrobielles Mittel in Zwischenschichträumen der Tonerde eingelagert wird und besagte Tonerde ein herbeigeführtes oder natürlich geschichtetes Gemisch aus Stevensit und Bentonit ist.

2. Zusammensetzung gemäß Anspruch 1, wobei besagtes antimikrobielles Mittel ein ätherisches Öl oder eine seiner natürlichen oder synthetischen flüchtigen Komponenten, vorzugsweise ein antiseptischer aromatischer Alkohol ist, der aus Thymol, Kresol, Carvacrol, Eugenol, Menthol und Zimtaldehyd und Isomeren, Derivaten und Gemischen davon ausgewählt ist.

3. Zusammensetzung gemäß einem der vorhergehenden Ansprüche, **dadurch gekennzeichnet, dass** besagtes flüchtiges antimikrobielles Mittel aus einem Gemisch aus zwei oder mehreren natürlichen oder synthetischen antimikrobiellen Mitteln besteht.

4. Zusammensetzung gemäß einem der vorhergehenden Ansprüche, **dadurch gekennzeichnet, dass** die essbare Tonerde ein Gemisch aus Ghassoul und Bentonit, vorzugsweise ein Gemisch aus marokkanischem Ghassoul und marokkanischem Bentonit ist, das vorzugsweise aus wenigstens 5 Gew.-% und besonders bevorzugt aus wenigstens 30 Gew.-% marokkanischem Ghassoul besteht.

5. Zusammensetzung gemäß einem der vorhergehenden Ansprüche, **dadurch gekennzeichnet, dass** besagtes flüchtiges antimikrobielles Mittel in einem Gewichtverhältnis zwischen 0,005 bis 0,33 bezogen auf besagte essbare Tonerde vorliegt.

6. Verfahren zur Herstellung besagter Zusammensetzung gemäß Ansprüchen 1 bis 5, **dadurch gekennzeichnet, dass** es die folgenden Schritte umfasst:
i) besagtes flüchtiges antimikrobielles Mittel wird in einem organischen oder anorganischen Lösemittel gelöst, um die antimikrobielle Lösung zu erhalten,
ii) die auf diese Weise erhaltene besagte antimikrobielle Lösung wird mit besagter essbarer Tonerde in Form von Pulver oder Pellets unter Rühr- und Temperaturbedingungen gemischt, die den Erhalt einer homogenen und stabilen Zusammensetzung erlauben, die aus einem Molekülkomplex besteht, der von besagtem flüchtigen antimikrobiellen Mittel durchsetzte essbare Tonerde enthält.
wobei vorzugsweise besagtes flüchtiges antimikrobielles Mittel aus Thymol, Kresol, Carvacrol, Menthol, Eugenol und Zimtaldehyd und Isomeren, Derivaten und Gemischen davon ausgewählt ist, besagtes organisches oder anorganisches Lösemittel ein pflanzliches Öl ist und besagte essbare Tonerde ein herbeigeführtes oder natürlich geschichtetes Gemisch aus Stevensit und Bentonit ist.

7. Verfahren zur Herstellung gemäß Anspruch 6, **dadurch gekennzeichnet, dass**:
i) eine Menge an flüchtigem antimikrobiellen Mittel portionsweise in einer Menge an pflanzlichem Öl gelöst, die 1 bis 20% der Menge an flüchtigem antimikrobiellen Mittel darstellt, gerührt und auf eine Temperatur von 30 bis 80°C erwärmt wird, um eine klare und homogene flüssige Lösung besagter antimikrobieller Lösung zu erhalten,
ii) eine Menge an antimikrobieller Lösung portionsweise durch Mischen in einer Menge an besagter pulverförmiger essbarer Tonerde eingeschlossen wird, die wenigstens die 3fache Menge der antimikrobiellen Lösung darstellt, um ein siebfähiges Pulver zu erhalten,
iii) das erhaltene siebfähige Pulver wird mittels Sieben sortiert, um die erwünschte Körnung zu erhalten.

8. Verfahren zur Herstellung gemäß Anspruch 6 oder 7, **dadurch gekennzeichnet, dass** besagtes antimikrobielles Mittel Thymol ist und besagtes organisches Lösemittel ein pflanzliches Öl ist und besagte essbare Tonerde nur marokkanischer Ghassoul oder ein Gemisch mit marokkanischem Bentonit ist.

9. Verfahren zur Herstellung gemäß Anspruch 8, **dadurch gekennzeichnet, dass** der Schritt des portionsweisen Lösens einer Menge an Thymol in einer Menge an pflanzlichem Öl, die 1 bis 20% der Menge an Thymol darstellt, bei einer Temperatur von 40 bis 60°C erfolgt.

10. Zusammensetzung gemäß einem der Ansprüche 1 bis 5, wobei besagte Zusammensetzung ein Futtermittelzusatz für Tiere ist.

11. Zusammensetzung gemäß Anspruch 10, wobei besagtes Tier aus Säugetieren, vorzugsweise aus Rindern, Schafen, Kaninchen, Schweinen, Ziegen und Pferden ausgewählt ist, aus Fischen, Bienen und Geflügel, vorzugsweise aus Masthühnern, Legehennen, Zuchthähnen und Zuchthennen, Perlhühnern, Puten, Wachteln, Enten, Gänsen und Tauben ausgewählt ist.

## Claims

1. A composition combining both antimicrobial and toxin-adsorption activities, **characterized in that** it is formed by a molecular complex obtained by mixing a volatile antimicrobial agent with an edible clay, said volatile antimicrobial agent being interspersed into the interfoliar space of the clay and said clay being a either caused or natural interlayered mixture of stevensite and bentonite.

2. The composition according to claim 1, **characterized in that** said antimicrobial agent is an essential oil or one of its volatile compounds, preferably an antiseptic aromatic alcohol selected from thymol, cresol, carvacrol, eugenol, menthol, cinnamaldehyde, natural or synthetic, and the isomers, derivatives and mixtures thereof.

3. The composition according to any one of the preceding claims, **characterized in that** said volatile antimicrobial agent is formed by a mixture of two or more of natural or synthetic antimicrobial agents.

4. The composition according to any one of the preceding claims, **characterized in that** said edible clay is a mixture of Ghassoul and bentonite, preferably Ghassoul from Morocco and bentonite from Morocco, preferably consisting of at least 5% by weight, and more preferably of at least 30% by weight, of Ghassoul from Morocco.

5. The composition according to any one of the preceding claims, **characterized in that** said volatile antimicrobial agent is present in a mass ratio from 0.005 to 0.33 based on said edible clay.

6. A method for manufacturing said composition according to claims 1 to 5, **characterized in that** it comprises the following steps:
i. said volatile antimicrobial agent is put into solution in an organic or mineral solvent in order to obtain the antimicrobial solution,
ii. said thereby obtained antimicrobial solution is mixed with said edible clay as a powder or pellets under stirring and temperature conditions giving the possibility of obtaining a homogenous and stable composition formed by a molecular complex containing said edible clay interspersed by said volatile antimicrobial agent,
preferably said volatile antimicrobial agent is selected from thymol, cresol, carvacrol, menthol, eugenol, cinnamaldehyde, and the isomers, derivatives and mixtures thereof, said organic or mineral solvent is a vegetable oil and said edible clay is a caused or natural interlayered mixture of stevensite and bentonite.

7. The manufacturing method according to claim 6, **characterized in that**:
i. an amount of volatile antimicrobial agent is gradually put into solution in an amount of vegetable oil, representing 1 to 20% of the amount of volatile antimicrobial agent, stirred and heated to a temperature ranging from 30°C to 80°C in order to obtain a homogenous and limpid liquid solution, a so called antimicrobial solution;
ii. an amount of the antimicrobial solution is gradually included by mixing in an amount of said powdered edible clay, representing at least three times the amount of antimicrobial solution, in order to obtain a sievable powder;
iii. the obtained sievable powder is calibrated by sieving in order to obtain the desired grain size.

8. The manufacturing method according to claim 6 or 7, **characterized in that** said antimicrobial agent is thymol, said organic solvent is a vegetable oil and said edible clay is Ghassoul from Morocco either alone or mixed with bentonite from Morocco.

9. The manufacturing method according to claim 8, **characterized in that** the step of gradually putting into solution an amount of thymol in an amount of vegetable oil representing 1 to 20% of the amount of thymol is conducted at a temperature ranging from 40°C to 60°C.

10. Composition according to any one of claims 1 to 5, said composition being a feed additive for an animal.

11. The composition according to claim 10, wherein said animal is selected from mammals, preferably selected from bovine animals, ovine animals, rabbits, pigs, caprine animals and equines, fish, bees and poultry, preferably selected from chicken broilers, laying hens, breeding roosters and hens, guinea fowls, turkeys, quails, ducks, geese and pigeons.
